# EUROPEAN PATENT APPLICATION

(11) **EP 4 317 409 A1**
(43) Date of publication of application: **07.02.2024**
(21) Application number: 22780271.7
(22) Date of filing: 18.03.2022
(51) Int. Cl.: C12M 3/04, B01D 39/00, C12M 3/06, C12N 5/071, C12N 5/0775, C12N 5/078, C12N 5/0789, C12N 5/09, C12N 5/095, C12Q 1/02, G01N 1/04

(54) **FILTER, MANUFACTURING METHOD THEREFOR, FILTER DEVICE, METHOD FOR SEPARATING OR FRACTIONATING RARE CELLS, AND METHOD FOR ANALYZING RARE CELLS IN CELL SUSPENSION**

(30) Priority: 30.03.2021 JP 2021058467
(71) Applicant: Toray Industries, Inc., Tokyo 103-8666 (JP); Public University Corporation Nara Medical University, Nara 634-8521 (JP)
(72) Inventor: SAWABATA, Noriyoshi, Kashihara-shi, Nara 634-8521 (JP); MORITA, Kohei, Kashihara-shi, Nara 634-8521 (JP); OBAYASHI, Chiho, Kashihara-shi, Nara 634-8521 (JP); SAKAGUCHI, Hirokazu, Otsu-shi, Shiga 520-8558 (JP); TOMINAGA, Yoshiaki, Otsu-shi, Shiga 520-8558 (JP); ARAKANE, Toru, Tokyo 103-8666 (JP); MORIOKA, Satoko, Otsu-shi, Shiga 520-8558 (JP); NODA, Yohei, Otsu-shi, Shiga 520-8558 (JP)
(74) Representative: Kador & Partner Part mbB
(86) International application number: PCT/JP2022/012883
(87) International publication number: WO 2022/210056

(57) **Abstract**

A filter having a plurality of filter pores penetrating one surface and the other surface of the filter, in which the filter pore has a first opening on the one surface and a second opening on the other surface, a ratio of a major axis diameter L1 to a minor axis diameter W1 of the first opening is 1.00 or more and 1.20 or less, the minor axis diameter W1 is 7.0 µm or more and 9.0 µm or less, a ratio of a major axis diameter L2 to a minor axis diameter W2 of the second opening is 1.00 or more and 1.20 or less, and a ratio of the minor axis diameter W2 to the minor axis diameter W 1 and a ratio of the major axis diameter L2 to the major axis diameter L1 are both 1.20 or more and 1.50 or less.

## Description

The present invention relates to a filter, a manufacturing method therefor, a filter device, a method for separating or fractionating rare cells, and a method for analyzing rare cells in a cell suspension.

### BACKGROUND ART

In the field of a physical examination to check for a specific disease, such as cancer screening, a liquid biopsy, in which a body fluid such as blood and saliva is used for a physical examination, is attracting increasing interest, from the viewpoint of reducing a cost and reducing a burden on a medical examinee. A technique for analyzing rare cells present in blood has attracted attention as one of liquid biopsies.

Examples of the rare cell include a circulating tumor cell (hereinafter referred to as "CTC"). CTC is a cell released from a primary tumor tissue or a metastatic tumor tissue and infiltrated into blood. CTCs in blood are expected to be analyzed and applied to grasp a pathological condition of a patient with an advanced solid cancer, determine a therapeutic effect, and the like. Further, it is also expected to culture living CTC cells and apply them to anticancer agent screening.

Various methods have been investigated for separating or fractionating rare cells such as CTCs. Examples thereof include a method using a cell sorter, a centrifugal separation method, and a method using a filter (hereinafter also referred to as a filter method). However, when using a cell sorter, cells can be fractionated with a high purity, but it is difficult to fractionate living cells. The centrifugal separation method is likely to be inferior in purity due to principle and technical factors. Therefore, the filter method is expected as a method capable of separating or fractionating living cells and separating or fractionating living cells at a relatively high purity.

For example, Patent Literature 1 discloses that a blood specimen is filtered using a filter having predetermined pores to separate or detect rare cells.

### CITATION LIST

### PATENT LITERATURE

Patent Literature 1: JP2016-180753A

### SUMMARY OF INVENTION

### TECHNICAL PROBLEM

However, in the filter method of related art, it is necessary to perform a pretreatment such as a hemolysis treatment on a specimen. In order to collect living rare cells, there is a demand for a separating or fractionating method that does not require a pretreatment such as a hemolysis treatment which may damage rare cells, in order to avoid damaging the rare cells as much as possible. Further, from the viewpoint of cost reduction, a simpler separating or fractionating method is required.

In view of the above problems, an object of the present invention is to provide a filter, a manufacturing method therefor, and a filter device capable of separating or fractionating rare cells without performing a pretreatment such as a hemolysis treatment. An object of the present invention is to provide a method for separating or fractionating rare cells using the above filter or filter device, and a method for analyzing rare cells in a cell suspension.

### SOLUTION TO PROBLEM

In order to solve the above problems, the present invention includes the following aspects.
1. A filter having a plurality of filter pores penetrating one surface and the other surface of the filter,
   in which
   the filter pore has a first opening on the one surface and a second opening on the other surface,
   a ratio (L1/W1) of a major axis diameter L1 to a minor axis diameter W1 of the first opening is 1.00 or more and 1.20 or less,
   the minor axis diameter W1 is 7.0 µm or more and 9.0 µm or less,
   a ratio (L2/W2) of a major axis diameter L2 to a minor axis diameter W2 of the second opening is 1.00 or more and 1.20 or less, and
   a ratio (W2/W1) of the minor axis diameter W2 to the minor axis diameter W1 and a ratio (L2/L1) of the major axis diameter L2 to the major axis diameter L1 are both 1.20 or more and 1.50 or less.
2. The filter according to above 1, having a thickness of 10 µm or more and 30 µm or less.
3. The filter according to above 1 or 2, in which a distance between a center of gravity C2 of the second opening and an intersection point C2' between the other surface and a perpendicular line drawn from a center of gravity C1 of the first opening to the other surface is 3 µm or less.
4. The filter according to any one of above 1 to 3, having a porosity rate of 10.5% or more and 25% or less.
5. The filter according to any one of above 1 to 4, in which the plurality of filter pores are arranged at equal intervals.
6. The filter according to any one of above 1 to 5, in which an interval between the filter pores is 8 µm or more and 30 µm or less.
7. The filter according to any one of above 1 to 6, being a filter including a film in which the filter pores are provided, the film having a total light transmittance of 80% or more.
8. The filter according to any one of above 1 to 7, being a filter including a film in which the filter pores are provided, the film having a Shore hardness of 30 or more and 50 or less and a Young's modulus of 5 MPa or more and 25 MPa or less.
9. The filter according to any one of above 1 to 8, including a thermoplastic resin.
10. The filter according to above 9, in which a main component of the thermoplastic resin is a polyethylene or a polypropylene.
11. A filter device including:
   the filter according to any one of above 1 to 10; and
   a holding portion configured to hold the filter, and
   the filter device being attachable to and detachable from a syringe.
12. A method for manufacturing the filter according to any one of above 1 to 10, the method including pressing a mold having a protrusion structure on a surface thereof against a film while heating the mold to form a filter pore.
13. A method for separating or fractionating rare cells in a cell suspension, the method including a filtration step of filtering a cell suspension using the filter according to any one of above 1 to 10 or the filter device according to above 11.
14. The method for separating or fractionating rare cells according to above 13, in which in the filtration step, the cell suspension is filtered due to an own weight thereof.
15. The method for separating or fractionating rare cells according to above 13 or 14, in which the rare cell is one or more cells selected from the group consisting of a cancer cell, a circulating tumor cell (CTC), an epithelial-mesenchymal transition CTC (EMTCTC), a clustered CTC, a vascular endothelial cell, a vascular endothelial precursor cell, a cancer stem cell, an epithelial cell, a hematopoietic stem cell, a mesenchymal stem cell, a fetal cell, and a combination thereof.
16. The method for separating or fractionating rare cells according to any one of above 13 to 15, in which in the filtration step, the cell suspension is filtered from a side of the one surface of the filter.
17. A method for analyzing rare cells in a cell suspension, the method including analyzing the rare cells separated or fractionated by the method according to any one of above 13 to 16 by a method including observing kinetics of the rare cells or measuring activity of the rare cells, or analyzing genes of the rare cells.

### ADVANTAGEOUS EFFECTS OF INVENTION

According to the present invention, rare cells can be separated or fractionated without subjecting a specimen to a pretreatment such as a hemolysis treatment which may damage the rare cells. That is, since damage to the rare cells in the specimen due to such a pretreatment can be prevented, it is expected that the rare cells can be separated or fractionated in a more intact state, and living cells can be easily fractionated. Further, since separating or fractionating of the rare cells becomes simpler, a cost can be reduced.

### BRIEF DESCRIPTION OF DRAWINGS

FIG. 1A, 1B, and 1C are schematic views schematically showing an example of a filter according to the present embodiment. FIG. 1A is a plan view seen from one surface side, FIG. 1B is a plan view seen from the other surface side, and FIG. 1C is a cross-sectional view taken along a line A-A in FIG. 1A and FIG. 1B.
FIG. 2 is a schematic view schematically showing a method of observing the filter using a confocal laser microscope.
FIG. 3A and 3B are showing images of the filter observed by the confocal laser microscope. FIG. 3A is showing an image obtained by observing reflected light from the other surface, and FIG. 3B is showing an image obtained by observing reflected light from an exposed portion of a reflector.
FIG. 4A and 4B are showing images obtained by binarizing the images in FIG. 3A and 3B. FIG. 4A is showing an image (an extracted image of a second opening) obtained by extracting a dark portion as a region corresponding to the second opening from FIG. 3A, and FIG. 4B is showing an image (an extracted image of a first opening) obtained by extracting a bright portion as a region corresponding to the first opening from FIG. 3B.
FIG. 5 is a flow diagram schematically showing an example of a method for manufacturing the filter according to the present embodiment.

### DESCRIPTION OF EMBODIMENTS

Hereinafter, embodiments of the present invention will be described with reference to the drawings, but the present invention is not limited to the following embodiments, and can be freely modified and implemented to the extent of without departing from the gist of the present invention. The embodiments described in the drawings are schematically illustrated to clearly describe the present invention, and do not necessarily accurately represent actual dimensions and scales.

### <Filter>

A filter according to the present embodiment has a plurality of filter pores penetrating one surface and the other surface of the filter, in which the filter pore has a first opening on the one surface and a second opening on the other surface, a ratio (L1/W1) of a major axis diameter L1 to a minor axis diameter W1 of the first opening is 1.00 or more and 1.20 or less, the minor axis diameter W1 is 7.0 µm or more and 9.0 µm or less, a ratio (L2/W2) of a major axis diameter L2 to a minor axis diameter W2 of the second opening is 1.00 or more and 1.20 or less, and a ratio (W2/W1) of the minor axis diameter W2 to the minor axis diameter W1 and a ratio (L2/L1) of the major axis diameter L2 to the major axis diameter L1 are both 1.20 or more and 1.50 or less.

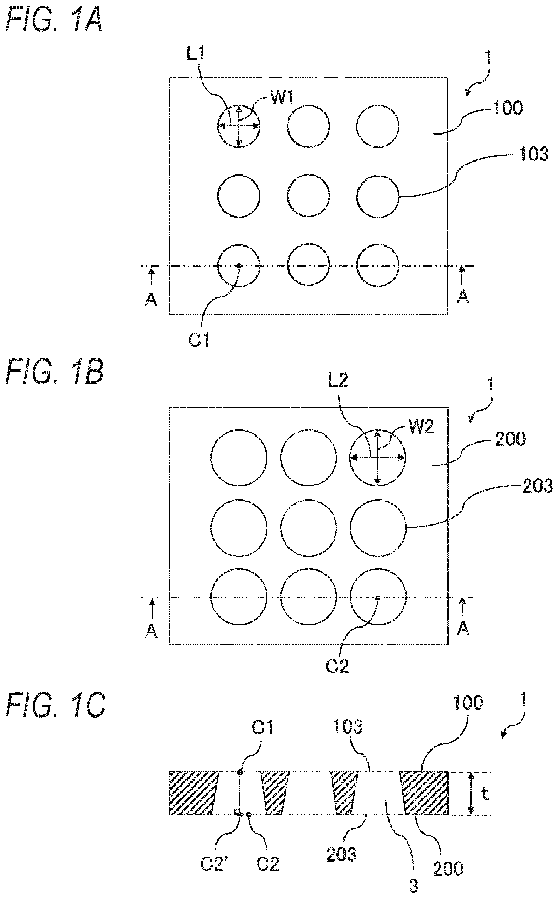

FIG. 1A, 1B, and 1C are schematic views schematically showing an example of the filter according to the present embodiment. FIG. 1A is a plan view seen from one surface side, FIG. 1B is a plan view seen from the other surface side, and FIG. 1C is a cross-sectional view taken along a line A-A in FIG. 1A and FIG. 1B. A filter 1 has a plurality of filter pores 3 penetrating one surface 100 and the other surface 200. The filter pores 3 each have a first opening 103 on the one surface 100 and a second opening 203 on the other surface 200.

In the first opening 103, the ratio (L1/W1) of the minor axis diameter W1 and the major axis diameter L1 is 1.00 or more and 1.20 or less. When (L1/W1) is 1.20 or less, the first opening tends to have a shape close to a perfect circle. (L1/W1) is preferably 1.18 or less, and more preferably 1.15 or less. (L1/W1) is 1.00 or more, and preferably exceeds 1.00 from the viewpoint of ease of manufacturing. It is preferred for the first opening to have a shape close to a perfect circle since rare cells of a specific size can be efficiently separated while maintaining a relatively large opening area.

The minor axis diameter W1 of the first opening 103 is 7.0 µm or more and 9.0 µm or less. When the minor axis diameter W1 is 7.0 µm or more, the rare cells of a specific size can be captured with a relatively small pressure loss. The minor axis diameter W1 is preferably 7.5 µm or more, and more preferably 8.0 µm or more. When the minor axis diameter W1 is 9.0 µm or less, the rare cells of a specific size can be captured without passing through. The minor axis diameter W1 is preferably 8.8 µm or less, and more preferably 8.5 µm or less.

In the second opening 203, the ratio (L2/W2) of the minor axis diameter W2 and the major axis diameter L2 is 1.00 or more and 1.20 or less. When (L2/W2) is 1.20 or less, the second opening tends to have a shape close to a perfect circle. (L2/W2) is preferably 1.18 or less, and more preferably 1.15 or less. (L2/W2) is 1.00 or more, and preferably exceeds 1.00 from the viewpoint of ease of manufacturing. It is preferred for the second opening to have a shape close to a perfect circle since the rare cells of a specific size can be efficiently separated while maintaining a relatively large opening area.

The ratio (W2/W1) of the minor axis diameter W1 and the minor axis diameter W2 and the ratio (L2/L1) of the major axis diameter L1 and the major axis diameter L2 are both 1.20 or more and 1.50 or less. The filter pore 3 satisfying such a requirement has a tapered shape that becomes larger from the first opening 103 to the second opening 203. Here, the tapered shape is a shape in which the second opening is larger than the first opening, that is, a shape satisfying the above requirement, and is not limited to a case where a diameter of the filter pore increases at a constant change rate with a distance in a thickness direction of the filter. When the filter pore 3 has such a tapered shape, a pressure loss when the specimen passes through the filter from the one surface 100 to the other surface 200 side can be prevented, as compared with a case where the filter pore has a cylindrical shape. From the viewpoint of sufficiently preventing the pressure loss, both (W2/W1) and (L2/L1) are 1.20 or more, preferably 1.22 or more, and more preferably 1.25 or more. From the viewpoint of preventing the filter pores from communicating with the adjacent filter pores, both (W2/W1) and (L2/L1) are 1.50 or less, preferably 1.45 or less, and more preferably 1.40 or less.

In the filter according to the present embodiment, the minor axis diameter and the major axis diameter of the opening are values measured by the following method.

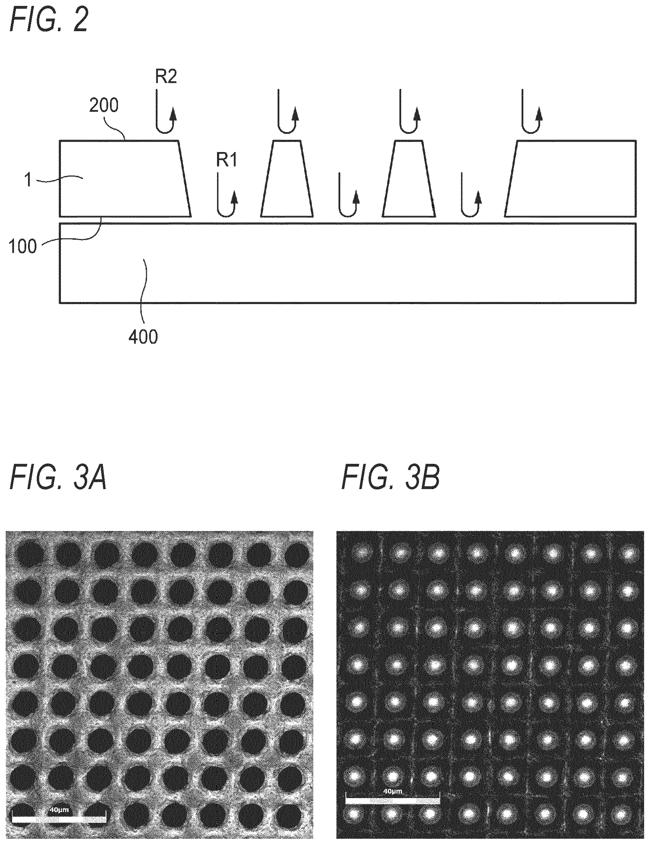

First, as schematically shown in FIG. 2, the filter 1 is disposed on a reflector 400 such that the one surface 100 faces the reflector 400. Next, the filter 1 is observed from the other surface 200 side using a confocal laser microscope (not shown). At this time, in a state where the filter 1 is fixed, reflected light R2 from the other surface 200 can be observed by focusing on the other surface 200 for observation. By focusing on a surface of the reflector 400 in contact with the one surface 100 for observation, reflected light R1 from an exposed portion of the reflector 400, that is, reflected light from a region having a shape corresponding to the shape of the first opening can be observed.

FIG. 3A and 3B are showing images of the filter observed by the confocal laser microscope. FIG. 3A is an image obtained by observing the reflected light from the other surface, and FIG. 3B is an image obtained by observing the reflected light from the exposed portion of the reflector.

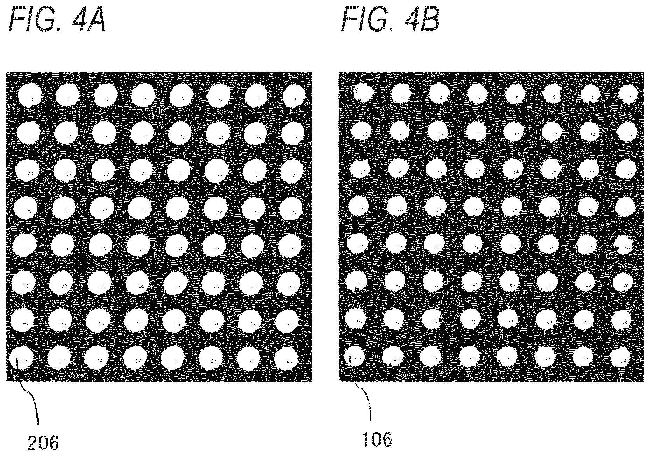

Next, the obtained observation image of the filter is binarized by image processing software. FIG. 4A and 4B show images obtained by binarizing the images in FIG. 3 A and 3B. FIG. 4A is an image (an extracted image of the second opening) obtained by extracting a dark portion as a region corresponding to the second opening from FIG. 3A. FIG. 4B is an image (an extracted image of the first opening) obtained by extracting a bright portion as a region corresponding to the first opening from FIG. 3B. By binarizing the images observed by the confocal laser microscope in this manner, an image in which substantially circular figures corresponding to the openings in the observation region are arranged is obtained.

A minimum value of a chord length passing through a center of gravity of a region 106 corresponding to the first opening in the extracted image of the first opening is defined as the minor axis diameter W 1 of the first opening. A maximum value of the chord length passing through the center of gravity of the region 106 is defined as the major axis diameter L1 of the first opening. Similarly, a minimum value of a chord length passing through a center of gravity of a region 206 corresponding to the second opening in the extracted image of the second opening is defined as the minor axis diameter W2 of the second opening. A maximum value of the chord length passing through the center of gravity of the region 206 is defined as the major axis diameter L2 of the second opening.

A specimen such as a cell suspension used for separating or fractionating the rare cells contains, for example, cells other than the rare cells, such as blood cells, and have a relatively high viscosity. Therefore, when the specimen is filtered using a filter of the related art without a pretreatment such as a hemolysis treatment, problems such as clogging of the filter occur, and thus separation of the rare cells is difficult. When the hemolysis treatment or the like is performed on the specimen as the pretreatment, the rare cells are damaged, and it may be difficult to fractionate the rare cells as living cells.

On the other hand, in the filter according to the present embodiment, for the plurality of filter pores, shapes of the respective filter pores and openings are adjusted to a specific range, and thus the pore diameters and the shapes of the plurality of filter pores are likely to be uniform. The filter according to the present embodiment was developed based on the finding that a specimen having a relatively high viscosity can be filtered without clogging by adjusting the pore shape to prevent the pressure loss while ensuring a separation performance by precisely and uniformly controlling the shape and the pore diameter of the opening. More specifically, the filter according to the present embodiment has an excellent separation performance since the first opening is adjusted to have a shape and a size suitable for separation of rare cells, and the filter pore has a tapered shape in which the size increases from the first opening toward the second opening, therefore the pressure loss during specimen filtration can be prevented, and the specimen can be filtered without the pretreatment such as a hemolysis treatment. According to the filter according to the present embodiment, since damage to the rare cells due to the pretreatment such as a hemolysis treatment can be prevented, the rare cells can be separated or fractionated in a more intact state, and living cells can be easily fractionated. Further, since the number of steps can be reduced in the process of separating or fractionating the rare cells, the cost can be reduced.

In addition to the above, in the filter of the related art, even when a pretreated specimen is used, it is necessary to filter the specimen by applying a pressure, such as applying a positive pressure with a syringe or the like or applying a negative pressure with a pump or the like. According to the filter according to the present embodiment, for example, by further adjusting a filter thickness, a porosity rate, a pore density, or the like to further prevent the pressure loss, the specimen can be filtered by natural falling due to an own weight of the specimen. It is preferred that the specimen can be filtered by natural falling since the process related to separating or fractionating of the rare cells is further simplified, and a load on the rare cells in the specimen can be further reduced.

Since the filter pore of the filter according to the present embodiment has a specific shape and size as described above, the filter is suitably used for separating or fractionating rare substances, preferably rare cells, in the specimen. By using the filter according to the present embodiment, preferably, by passing the specimen from one surface side to the other surface side for filtration, when the specimen contains desired rare cells (rare substances), the rare cells (the rare substances) remain on the one surface of the filter after the filtration, and are separated or fractionated. In the filter according to the present embodiment, in particular, since the minor axis diameter W1 of the first opening is 7.0 µm or more and 9.0 µm or less, and the first opening has a shape close to a perfect circle, blood cells or the like having a size of about 2 µm to 8 µm can pass through the filter pore, and rare cells (rare substances) having a size of about 9 µm to 30 µm can be prevented from passing through the filter pore, so that the rare cells (the rare substances) can be efficiently separated or fractionated. As will be described in detail later, examples of such rare cells include a cancer cell such as a circulating tumor cell (CTC), a clustered CTC, and an epithelial-mesenchymal transition CTC (EMTCTC), a vascular endothelial cell, a vascular endothelial precursor cell, a cancer stem cell, an epithelial cell, a hematopoietic stem cell, a mesenchymal stem cell, and a fetal cell. That is, the filter according to the present embodiment is particularly suitably used for separating or fractionating a cancer cell such as a circulating tumor cell (CTC), a clustered CTC, and an epithelial-mesenchymal transition CTC (EMTCTC), a vascular endothelial cell, a vascular endothelial precursor cell, a cancer stem cell, an epithelial cell, a hematopoietic stem cell, a mesenchymal stem cell, and a fetal cell.

Hereinafter, a preferred aspect of the filter according to the present embodiment will be further described.

A thickness t of the filter according to the present embodiment is preferably 10 µm or more and 30 µm or less. The thickness t is preferably 10 µm or more, more preferably 15 µm or more, and even more preferably 18 µm or more from the viewpoint of handleability. The thickness t is preferably 30 µm or less, more preferably 25 µm or less, and even more preferably 23 µm or less from the viewpoint of further preventing the pressure loss. The thickness t can be measured by a measuring instrument such as a slide calipers or a micrometer.

A distance between a center of gravity C2 of the second opening 203 and an intersection point C2' between the other surface 200 and a perpendicular line drawn from a center of gravity C1 of the first opening 103 to the other surface 200 is preferably 3 µm or less. The distance between C2' and C2 is more preferably 2 µm or less, and even more preferably 1 µm or less. A small distance between C2' and C2 means that the filter pore penetrates in a direction more parallel to the thickness direction of the filter (with a smaller inclination). It is preferred that the filter pore penetrates more in parallel to the thickness direction of the filter since the pressure loss can be further prevented. It is preferred since the image becomes easy to see when the filter after specimen filtration is observed with a microscope or the like.

FIG. 1C schematically shows a relationship among C1, C2', and C2. In FIG. 1C, an arrangement is adjusted such that C2' and C2 are clearly shown at different positions for convenience of explanation, but C2' and C2 may be at the same position, that is, a distance between C2' and C2 may be 0 µm.

In the filter according to the present embodiment, the distance between C2' and C2 of each filter pore is obtained, for example, by comparing a coordinate of a center of gravity of each region 206 on the image in the extracted image of the second opening described above with a coordinate of a center of gravity of each region 106 corresponding to the first opening on the image in the extracted image of the first opening, and converting the distance into a distance on the filter.

A porosity rate of the filter according to the present embodiment is preferably 10.5% or more and 25% or less. When the porosity rate is 10.5% or more, the pressure loss is more easily prevented. When the porosity rate is low, an operation of applying a pressure such as extrusion or suction of a liquid to be treated is likely to be required, which may result in damage to rare cells or a collecting loss due to slipping of an object to be fractionated. On the other hand, by setting the porosity rate to 10.5% or more, the filter can be suitably used for a filter method capable of a natural falling. From the same viewpoint, the porosity rate is more preferably 12% or more, and even more preferably 18% or more. The higher porosity rate is preferable, but from the viewpoint of maintaining the mechanical strength of the filter, the porosity rate is preferably 25% or less. The porosity rate is more preferably 23% or less, and even more preferably 22% or less.

In the filter according to the present embodiment, the porosity rate is a value obtained by the following equation: porosity rate (%) = (first opening area/filter area) × 100.

A pore density of the filter according to the present embodiment is preferably 3600/mm² or more and 4200/mm² or less. When the pore density is 3600/mm² or more, the pressure loss is more easily prevented. The pore density is more preferably 3700/mm² or more, and even more preferably 3800/mm² or more. It is preferred that the pore density is 4200/mm² or less since an area of a non-opening of the filter increases, and the strength of the filter can be maintained. The pore density is more preferably 4100/mm² or less, and even more preferably 4000/mm² or less.

In the filter according to the present embodiment, the pore density is a value obtained by the following equation: pore density (number/mm²) = (number of filter pores (number)/filter area (mm²)).

In the filter according to the present embodiment, the pore density in any region of 0.64 mm × 0.64 mm is preferably 3600/mm² or more and 4200/mm² or less. The pore density in any region of 0.64 mm × 0.64 mm is more preferably 3700/mm² or more, and even more preferably 3800/mm² or more. The pore density in any region of 0.64 mm × 0.64 mm is more preferably 4100/mm² or less, and even more preferably 4000/mm² or less. By satisfying such a requirement, the distribution of the filter pores on the filter surface is likely to be uniform. When the distribution of the filter pores on the filter surface is uniform, occurrence of deviation in a flow of the specimen during the specimen filtration can be prevented. By preventing the deviation of the flow of the specimen, occurrence of clogging during separation and separation of cells in an aggregated state, which may cause a problem during culture or analysis, can be prevented.

From the same viewpoint as described above and from the viewpoint of preventing communication between the plurality of filter pores during or after manufacturing, it is more preferred that the plurality of filter pores of the filter according to the present embodiment are arranged at equal intervals. A specific arrangement pattern of the filter pores is not particularly limited, and is preferably, for example, a pattern in which a predetermined shape or a figure is repeatedly arranged at a predetermined period. Specific examples thereof include a grid shape such as a square grid shape, a triangular grid shape, a hexagonal grid shape, and a parallel grid shape, a spiral shape, a concentric circular shape, and a radial shape. The expression "filter pores are arranged at equal intervals" does not mean that all intervals between the filter pores are exactly equal. The equal intervals means substantially equal intervals within a range in which the effect of the present invention is exhibited.

The interval between the filter pores (the interval of the filter pores) is preferably 8 µm or more and 30 µm or less. When the interval between the filter pores is in the above range, the pore density and the porosity rate can be easily adjusted to the above preferred ranges. It is preferred that the interval between the filter pores is equal to or more than the above lower limit value since the plurality of filter pores can be prevented from communicating with each other during manufacturing or after manufacturing. The interval between the filter pores is more preferably 10 µm or more, and even more preferably 14 µm or more. The interval between the filter pores is more preferably 28 µm or less, and even more preferably 24 µm or less. It is more preferred that the plurality of filter pores are arranged at substantially equal intervals and the intervals are within the above range.

The interval between the filter pores (the interval of the filter pores) refers to a distance from a center of gravity of the first opening of one filter pore to a center of gravity of the first opening of another filter pore adjacent to the one filter pore.

Components constituting the filter according to the present embodiment are not particularly limited, and the filter preferably contains a thermoplastic resin from the viewpoint of easily obtaining a filter including the preferred aspect described above. A proportion of the thermoplastic resin in the filter is preferably 60 mass% or more, and more preferably 80 mass% or more. An upper limit of the proportion of the thermoplastic resin is not particularly limited, and 100 mass% is a substantial upper limit.

Specific preferred examples of the main component of the thermoplastic resin include a polyolefin-based resin such as a polyethylene, a polystyrene, a polypropylene, a polyisobutylene, a polybutene, and a polymethylpentene. These thermoplastic resins are preferred from the viewpoint of excellent releasability during manufacturing and excellent moldability. The main component refers to a component occupying for 50 mass% or more of the entire thermoplastic resin contained in the filter as 100 mass%. The main component is preferably 50 mass% or more, and more preferably 80 mass% or more. An upper limit value is not particularly limited, and 100 mass% is a substantial upper limit.

The main component of the thermoplastic resin is preferably a polyethylene or a polypropylene. By using a polyethylene or a polypropylene, a through hole can be formed at a relatively low temperature, and thus productivity is easily improved. It is preferred since the control of the pore diameter is easy and the filter has moderate flexibility and transparency, and thus it is easy to obtain the filter including the preferred aspect described above.

The filter may contain, in addition to the thermoplastic resin, various additives added for various purposes such as manufacturing or use. Examples of such additives include organic fine particles, inorganic fine particles, a dispersant, a dye, a fluorescent whitening agent, an antioxidant, a weathering agent, an antistatic agent, a releasing agent, a thickening agent, a plasticizer, a pH adjusting agent, and a salt. In particular, it is preferred to add a small amount of a carboxylic acid having a low surface tension, such as a long-chain carboxylic acid or a long-chain carboxylate, or a derivative thereof, or an alcohol compound having a low surface tension, such as a long-chain alcohol, a derivative thereof, or a modified silicone oil at a time of polymerization, as a releasing agent during manufacturing.

The components constituting the filter can be specified by general chemical analysis such as infrared spectroscopy (IR) or nuclear magnetic resonance (NMR).

A configuration of the filter according to the present embodiment is not particularly limited, and for example, a filter obtained by providing the filter pores described above in a film is preferred. A material of the film is not particularly limited. For example, the film is preferably a resin film, and more preferably a resin film containing a thermoplastic resin, from the viewpoint of easily obtaining the filter including the preferred aspect described above. A more preferred aspect of the material of the film is specifically the same as the preferred aspect of the components constituting the filter described above. As long as the effect of the present invention is not impaired, the film may be a layer made of a single resin or a laminate made of a plurality of resin layers. Various coatings may be applied to the film, if necessary.

A total light transmittance of the film is preferably 80% or more. The total light transmittance is more preferably 82% or more, and even more preferably 85% or more. When the total light transmittance is in the above range, the filter after the specimen filtration is easy to be used for microscopic observation or living cell culture. An upper limit of the total light transmittance is not particularly limited, and the total light transmittance is typically 100% or less.

The total light transmittance can be measured by a method for measuring a total light transmittance (JIS K 7361-1) using a single beam photometry device.

A Shore hardness of the film is preferably 30 or more and 50 or less. It is preferred that the Shore hardness of the film is 30 or more since handleability of the filter with a tweezer or the like is improved. The Shore hardness is more preferably 32 or more, and even more preferably 35 or more. It is preferred that the Shore hardness is 50 or less since the filter has moderate flexibility, and the pressure loss is easily prevented. The Shore hardness is more preferably 48 or less, and even more preferably 46 or less.

The Shore hardness can be measured by a method of a durometer hardness test (JIS K 7215).

A Young's modulus of the film is preferably 5 MPa or more and 25 MPa or less. It is preferred that the Young's modulus of the film is 5 MPa or more since deformation of an opening shape of the filter is easily prevented during filtration. The Young's modulus is more preferably 7 MPa or more, and even more preferably 10 MPa or more. It is preferred that the Young's modulus is 25 MPa or less since the filter has moderate flexibility, and the pressure loss is more easily prevented. The Young's modulus is more preferably 22 MPa or less, and even more preferably 20 MPa or less.

The Young's modulus can be measured by a method of a tensile property test (JIS K 7161).

From the viewpoint of handleability and filterability, it is more preferred that both the Shore hardness and the Young's modulus are in the above ranges.

### <Filter Device>

The present invention also relates to a filter device that is attachable to and detachable from a syringe and includes the filter according to the present embodiment and a holding portion that holds the filter. A shape and the like of the holding portion are not particularly limited as long as the holding portion can hold the filter, and the holding portion can be appropriately designed according to an application, a syringe to be attached and detached, and the like. A mechanism for attaching to and detaching from the syringe is not particularly limited, and can be adjusted according to the application, the syringe to be attached and detached, and the like. The filter device according to the present embodiment is preferred since in the specimen filtration using the filter according to the present embodiment, an existing syringe or the like which is easily available as a flow path of the specimen can be used, and filtration can be performed more easily.

### <Method for Separating or Fractionating Rare Cells>

The filter according to the present embodiment or the filter device according to the present embodiment is suitably used for separating or fractionating rare cells. That is, the present invention also relates to a method for separating or fractionating rare cells in a specimen. Examples of such a method include a method including a filtration step of filtering a specimen using the filter according to the present embodiment or the filter device according to the present embodiment.

In the filtration step, the specimen is preferably filtered from the one surface of the filter. Filtration of the specimen from the one surface means filtration by passing the specimen from one surface side to the other surface side of the filter. When the specimen contains desired rare cells, the rare cells remain on the one surface of the filter after filtration, and thus the rare cells are separated or fractionated.

In the filtration step, a pressure difference may be provided between the one surface side and the other surface side of the filter. From the viewpoint of preventing damage to the rare cells in the specimen, the pressure difference is preferably 10 Pa or less, and more preferably 1 Pa or less. It is more preferred that no pressure difference is provided in the filtration step, that is, filtration is performed by an own weight of the specimen. In the filter according to the present embodiment, since the pressure loss during filtration can be prevented, the pressure difference can be made relatively small, and further, filtration can be performed by an own weight of the specimen.

A liquid feed rate of the filter is not particularly limited, and is, for example, preferably 5 nl/min or more, more preferably 10 nl/min or more, and even more preferably 20 nl/min or more per filter pore from the viewpoint of reducing a time required for filtration. From the viewpoint of reducing a load on a filtration target, for example, the liquid feed rate is preferably 1000 nl/min or less, more preferably 500 nl/min or less, and even more preferably 100 nl/min or less. As described above, a driving force such as a pressure difference is not necessarily required for liquid feeding, and the specimen may be fed at the above rate by the own weight of the specimen.

A filtration area is not particularly limited, and can be set in consideration of, for example, the number of filter pores and a filtration amount of the specimen. From the viewpoint of increasing filtering capacity, the filtration area is preferably 50 mm² or more, more preferably 100 mm² or more, and even more preferably 130 mm² or more. From the viewpoint of reducing a required amount of the specimen, the filtration area is preferably 1000 mm² or less, more preferably 800 mm² or less, and even more preferably 500 mm² or less.

The specimen that can be filtered by the filter according to the present embodiment is not particularly limited, and for example, a cell suspension is preferred. Specific examples of the cell suspension include blood, urine, saliva, cerebrospinal fluid, pleural fluid, ascitic fluid, and a residual specimen after cell diagnosis.

According to the filter of the present embodiment, a filtration treatment can be performed without performing the pretreatment such as a hemolysis treatment on the specimen. Accordingly, from the viewpoint of preventing damage to the rare cells and facilitating the fractionating of living cells, it is preferred not to perform, on the specimen, the pretreatment such as a hemolysis treatment which may damage the rare cells. However, this does not preclude such a treatment depending on the purpose, use, or the like. A known pretreatment such as dilution of a specimen or a labeling treatment of rare cells may be performed depending on the purpose or use, a state of the specimen, or the like.

Examples of the substance separated by the filter according to the present embodiment include a rare substance contained in the above specimen, and examples of the rare substance include rare cells and rare substances. As the substance to be separated, rare cells are typically preferred. In the embodiment described above, the rare cells are described as an example of the substance to be separated, but the substance to be separated is not limited to the rare cells as described above.

Examples of the rare cell include one or more cells selected from the group consisting of a cancer cell, a circulating tumor cell (CTC), an epithelial-mesenchymal transition CTC (EMTCTC), a clustered CTC, a vascular endothelial cell, a vascular endothelial precursor cell, a cancer stem cell, an epithelial cell, a hematopoietic stem cell, a mesenchymal stem cell, a fetal cell, and a combination thereof. The rare cell is preferably one or more cells selected from the group consisting of a circulating tumor cell (CTC), an epithelial-mesenchymal transition CTC (EMTCTC), and a clustered CTC. Since the shape and size of the pore of the filter according to the present embodiment are adjusted to a specific range, the filter is suitably used for separating or fractionating the rare cells.

Examples of the rare substance include asbestos, a cholesterin crystal, a thrombus, a ureate crystal, and a calcium pyrophosphate crystal.

### <Method for Analyzing Rare Cells in Cell Suspension>

The present invention also relates to a method for analyzing rare cells in a cell suspension. That is, the method for analyzing rare cells in a cell suspension according to the present embodiment includes analyzing the rare cells separated or fractionated by the method for separating or fractionating rare cells according to the present embodiment by a method including observing kinetics of the rare cells or measuring activity of the rare cells, or analyzing genes of the rare cells. That is, according to the filter of the present embodiment, since the rare cells can be separated or fractionated as living cells, it is possible to analyze by the method including observing kinetics of the rare cells or measuring activity of the rare cells, or to analyze genes of the rare cells. The analysis method is not particularly limited, and a known analysis technique can be used. Depending on the specific technique, the analysis may be performed with the rare cells, which are separated or fractionated by remaining on the one surface of the filter after filtration, in a state where the rare cells remain on the filter, or with the rare cells collected from the filter.

### <Method for Manufacturing Filter>

The method for manufacturing a filter according to the present embodiment is not particularly limited as long as the filter described above can be obtained, and examples thereof include a method including pressing a mold having a protrusion structure on a surface thereof against a film while heating the mold to form a filter pore (a filter pore forming step). Hereinafter, a preferred example of the method for manufacturing a filter according to the present embodiment will be specifically described with reference to the drawings.

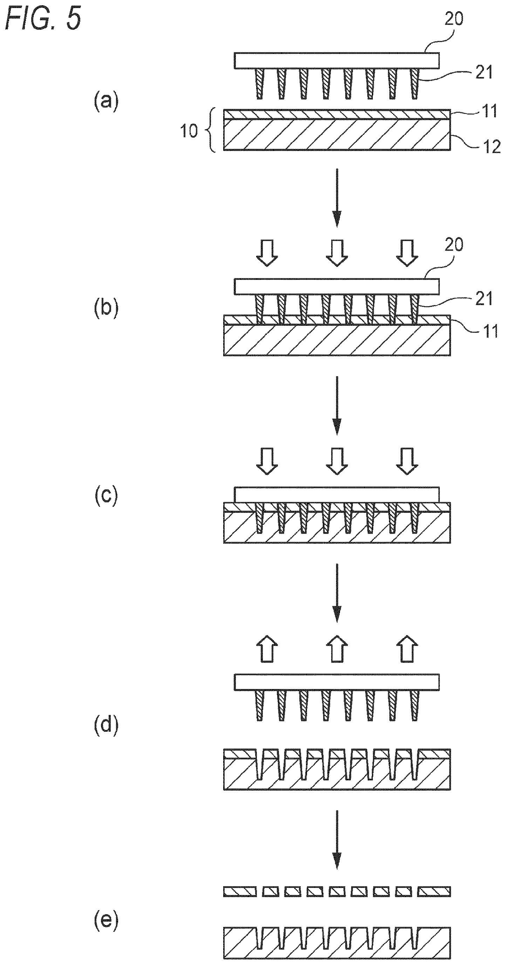

FIG. 5 is a flow diagram schematically showing an example of the method for manufacturing a filter according to the present embodiment. In the above filter pore forming step, the film is preferably used in a state of a laminated structure 10 in which at least a film 11 and a support film 12 are laminated. That is, in the filter pore forming step, it is preferred to form a through hole in the film 11 and to form a recess communicating with the through hole in the support film 12 by pressing a mold 20 having protrusion structures 21 on a surface thereof against the laminated structure 10 on a film 11 side of the laminated structure 10 while heating the mold 20. Here, the through hole formed in the film 11 corresponds to the filter pore of the filter according to the present embodiment.

When the laminated structure is used in the filter pore forming step, the film and the support film are preferably peeled off after the filter pore forming step (a peeling step). By the peeling step, the film having the through hole (the filter pores) formed in the filter pore forming step is obtained as a filter.

More specifically, an example of the method for manufacturing a filter according to the present embodiment is as follows.

First, as shown in (a) of FIG. 5, the laminated structure 10 in which the film 11 and the support film 12 are laminated and the mold 20 in which independent protrusion structures are arranged at predetermined positions on the surface thereof are prepared. Here, the film 11 preferably contains a thermoplastic resin P1, and the support film preferably contains a thermoplastic resin P2. A melting point of the thermoplastic resin P1 is defined as Tm1, and a glass transition point of the thermoplastic resin P2 is defined as Tg2.

As for a proportion of each thermoplastic resin contained in each layer, it is preferred that the thermoplastic resin is contained in 60 mass% or more when the entire layer is taken as 100 mass%. Further, the proportion of each thermoplastic resin is more preferably 80 mass% or more. In addition to the thermoplastic resin P1 or the thermoplastic resin P2, an additive or a coating component for imparting moldability or releasability may be contained in each layer. An upper limit value is not particularly limited, and a substantial upper limit is 100 mass%.

An interface between the film and the support film is preferably peelable, and the interface between the film and the support film is preferably laminated using an action of an adhesive formed by coating or the like. Although a two-layer laminated structure of the film and the support film is described in the present embodiment, another layer may be provided on a side opposite to the film with interposing the support film therebetween. It is preferred to apply a material having a composition same as that of the film to coating of the surface of the film since the flatness after molding is improved.

The laminated structure refers to a structure in which two or more layers containing different components are laminated. The laminated structure may be a continuous film conveyed in a roll-to-roll manner, or may be a single sheet.

A glass transition temperature is a temperature at which tanδ becomes maximum when temperature dependence (temperature dispersion) is measured according to a method described in JIS K 7244-4 (1999) with a sample dynamic amplitude speed (a driving frequency) of 1 Hz, a tensile mode, a distance between chucks of 5 mm, and a temperature rising rate of 2°C/min.

The melting point referred to herein is a melting point Tm during a temperature rising process (a temperature rising rate: 20°C/min), which is obtained by differential calorimetry (DSC), and a temperature at the peak top of a crystal melting peak of 2nd Run, which is obtained by heating a resin from 25°C to 300°C at a temperature rising rate of 20°C/minute (1stRUN), holding in that state for 5 minutes, then rapidly-quenching to 25°C or lower, and again increasing the temperature from room temperature to 300°C at a temperature rising rate of 20°C/minute, by a method based on JIS K 7121 (1999) same as described above, is defined as the melting point of the resin.

In the example of the manufacturing method according to the present embodiment, first, the mold 20 having the protrusion structures 21 on the surface thereof is heated. The heating is preferably performed such that the temperature of the mold is in a range of Tm1 or higher and Tg2 or higher. The heating may be performed in a state where the mold and the laminated structure are in contact with each other. By keeping the mold and the laminated structure in contact with each other, the flatness of the laminated structure can be maintained in a good state.

An upper limit value of the heating temperature of the mold is not limited, and the heating temperature is preferably equal to or lower than a thermal decomposition point of the thermoplastic resin P1 and equal to or lower than a thermal decomposition point of the thermoplastic resin P2.

Next, as shown in (b) of FIG. 5, the mold 20 is pressed against the surface of the film 11 of the heated laminated structure 10 such that the protrusion structure surface comes into contact therewith. When the protrusion structure 21 has a proper height, the protrusion structure penetrates through the film 11 and pierces the support film 12 by pressuring. Then, as shown in (c) of FIG. 5, the mold 20 and the laminated structure 10 are brought into contact with each other without a gap.

A pressure and a pressurizing time required at this time depend on the material of the film, a transfer shape, particularly an aspect ratio of the unevenness. Generally, the pressing pressure is preferably in a range of 1 MPa to 50 MPa, and the molding time is preferably in a range of 0.01 seconds to 60 seconds.

The pressing pressure is more preferably in a range of 2 MPa to 40 MPa, and even more preferably in a range of 3 MPa to 15 MPa. The molding time is more preferably in a range of 1 second to 50 seconds, and even more preferably in a range of 3 seconds to 30 seconds.

The mold 20 may be pressed against the laminated structure 10 by position control. That is, the mold 20 may be moved to a predetermined position and pressed against the laminated structure 10. The predetermined position is a position where a flat surface of the mold including the protrusion structures can abut on the surface of the film without a gap.

After the pressure is increased, the pressure may be removed while maintaining the position of the mold to maintain the contact between the mold 20 and the laminated structure 10.

Next, as shown in (c) of FIG. 5, the mold is cooled while maintaining the pressurized state or the contact state. It is preferred to cool down to equal to or lower than the glass transition temperature Tg2 of the thermoplastic resin P2 constituting the support film. It is preferred to cool down to Tg2 or lower since resin deformation after the mold 20 is peeled off from the laminated structure 10 can be prevented, and the through holes can be formed with high accuracy.

Next, as shown in (d) of FIG. 5, the laminated structure 10 is peeled off from the mold 20. The peeling is performed by moving the mold or the laminated structure apart in a direction perpendicular to the surface of the laminated structure. In the case where the laminated structure is a continuous film, it is preferred to continuously apply tension in the direction perpendicular to the surface of the laminated structure such that a linear peeling position moves continuously for peeling. The support film may be peeled off immediately before using the film having the through holes, that is, the manufactured filter, while maintaining this state. It is preferred since the support film serves as a cover film, and when the support film is peeled off immediately before use, the surface is less likely to be damaged, and the film can be treated as a thick film having high rigidity immediately before use, and thus the workability is good.

In (e) of FIG. 5, the film 11 is peeled off from the support film 12. The peeling is preferably performed by applying tension to the film or the support film in the direction perpendicular to the surface of the film or the support film such that a linear peeling position moves continuously for peeling, from the viewpoint of preventing peeling marks.

By performing the steps described above with reference to FIG. 5, a filter obtained by forming the filter pores whose shape can be controlled with high accuracy in the film 11 can be manufactured. According to the above manufacturing method, since the film is in a molten state during molding, the film when pressed against the protrusion structure undergoes plastic deformation with a behavior similar to that of a viscous material, and the through hole is formed with less burr on the opening end surface. Further, when a protrusion pattern (the protrusion structure) is pushed in, the support film undergoes viscoelastic deformation, and the protrusion structure can smoothly enter the support film, so that even at an interface between the film and the support film, a clean end surface with less burrs can be formed.

In the method as described above, by adjusting a shape and an arrangement of the protrusion structure of the mold in accordance with the shape of the filter pore, a filter in which a shape, an arrangement, and the like of the filter pore and an opening thereof are controlled with high accuracy can be manufactured. That is, in the manufacturing method according to the present embodiment, a desired filter can be manufactured by adjusting the shape and arrangement of the protrusion structure of the mold so as to correspond to a preferred aspect of the shape and arrangement of the filter pore of the filter according to the present embodiment.

A material of the mold is preferably a metal having high-strength and high-thermal conductivity, for example, nickel, steel, stainless steel, or copper. An outer surface thereof may be plated to improve workability.

The height, shape, cross-sectional shape, arrangement, and the like of the protrusion structure are appropriately determined depending on a required shape of the filter pore and a required thickness of the film. The height of the protrusion structure is preferably long enough to penetrate the thickness of the film 11. That is, it is preferred that the protrusion structure has a height to penetrate the film 11 when the mold 20 is in close contact with the laminated structure 10 during molding. The shape of the protrusion structure is preferably a shape corresponding to the filter pore shape described above, for example, a truncated cone shape, or a conical shape. A cross-sectional shape of a cross-section perpendicular to a height direction of the protrusion structure is preferably circular, for example.

Examples of the method for producing a mold having a protrusion structure on a surface thereof include a method in which a metal surface is directly subjected to cutting, laser processing, or electron beam processing, and a method in which a plating film formed on a metal surface is directly subjected to cutting, laser processing, or electron beam processing. Examples thereof include a method in which after a resist is applied onto a substrate, a photolithography method is used to form the resist in a predetermined patterning, then the substrate is etched to form a recess, and a reverse pattern of the recess is obtained by electroforming after resist removal. A conical pattern can be obtained by applying anisotropic etching. As the substrate, a silicon substrate or the like can be applied in addition to the metal plate.

As for the material or the like of the film 11, a preferred aspect is same as the preferred aspect of the film in the filter described above.

Specific preferred examples of the main component of the thermoplastic resin constituting the support film 12 include a polyester-based resin such as a polyethylene terephthalate, a polyethylene-2,6-naphthalate, a polypropylene terephthalate, and a polybutylene terephthalate, a polyolefin-based resin such as a polyethylene, a polystyrene, a polypropylene, a polyisobutylene, a polybutene, and a polymethylpentene, a polyamide-based resin, a polyimide-based resin, a polyether-based resin, a polyester amide-based resin, a polyether ester-based resin, an acrylic resin, a polyurethane-based resin, a polycarbonate-based resin, a cycloolefin polymer, and a polyvinyl chloride-based resin. Particularly preferred are a polymethyl methacrylate and a cycloolefin polymer. The main component refers to a component occupying for 50 mass% or more of the entire resin constituting the support film as 100 mass%. The main component is preferably 50 mass% or more, and more preferably 80 mass% or more. The support film 12 may appropriately contain various additives similar to the components constituting the filter.

The thermoplastic resin P2 is preferably a polymethyl methacrylate, a cycloolefin polymer, or a polycarbonate. Particularly preferred are a polymethyl methacrylate and a cycloolefin polymer. By using a polymethyl methacrylate, a cycloolefin polymer, or a polycarbonate, the recess communicating with the through hole (the filter pore) can be formed with high accuracy.

The film or the support film may be a layer composed of a single body of the resin described above or a laminate composed of a plurality of resin layers as long as the effects of the present invention are not impaired. In this case, surface properties such as releasability and friction resistance can be imparted compared to a single layer. Even in the case of a laminate composed of a plurality of resin layers as described above, it is preferred that the thermoplastic resin component mainly satisfies the above-described requirements in each of the film and the support film.

As the method for manufacturing the film and the support film, for example, it is preferred to form the thermoplastic resin into a film by melt extrusion. When a release layer, an adhesive layer, or the like is provided on the surface layer, a method of coextrusion and processing into a film shape may be used, and the layer may be provided by coating after film formation. Alternatively, a method of extrusion laminating a surface layer raw material onto a film made of a single film may be used. For the lamination of the film and the support film, a method of laminating by pinching and pressing with a roller, a method of heat laminating with a heated roller, or the like can be applied.

### Examples

Hereinafter, Examples of the present invention will be specifically described, but the present invention is not limited thereto.

### [Filter Evaluation]

### (Shape and Arrangement of Filter Pores)

### (Observation with Confocal Laser Microscope)

A self-adhesive film (material: a polyethylene, manufactured by Toray Advanced Film Co., Ltd., Model No.: TORETEC 7721) was used as a reflector, and each filter was placed on the reflector such that one surface faces down. Next, the filter was observed from the other surface side using a confocal laser microscope (manufactured by Olympus Corporation, Model No.: OLS 4100). At this time, in a state where the filter was fixed, an image observed by focusing on the other surface of the filter and an image observed by focusing on a surface of the reflector in contact with the one surface of the filter were obtained. An observation range was 131 µm square, and an irradiation intensity was adjusted in this range such that a reflected light intensity during laser observation did not exceed a maximum intensity of a light receiving element.

In a filter in Comparative Example 1, since a thickness of the filter was large, reflected light of a laser could not be obtained from the surface of the reflector, and thus image observation using a confocal laser microscope was difficult. Therefore, the filter in Comparative Example 1 was observed by acquiring a cross-sectional image of the filter with a scanning electron microscope.

### (Binarization)

Next, the obtained observation image of the filter is binarized by image processing software. For the image observed by focusing on the other surface of the filter, a portion having a reflected light intensity of 0% to 10% with respect to a maximum value of the reflected light intensity in the observation image was extracted as a dark portion. At this time, a portion on an imaging region boundary and a portion with 100 pixels or less were excluded from the target. The entire image is imaged with 1024 × 1024 pixels, and a portion with 100 pixels or less is 1.6 µm² or less in terms of area. For the image observed by focusing on the surface of the reflector in contact with the one surface of the filter, a portion having a reflected light intensity of 13% to 100% with respect to a maximum value of the reflected light intensity in the observation image was extracted as a bright portion. At this time, a portion on an imaging region boundary and a portion with 2000 pixels or less were excluded from the target. The entire image is imaged at 1024 × 1024 pixels, and a portion with 2000 pixels or less is 32 µm² or less in terms of area.

From the binarized images, the minor axis diameter W1 and the major axis diameter L1 of the first opening, the minor axis diameter W2 and the major axis diameter L2 of the second opening, a distance between C2' and C2, a porosity rate, a pore density, and an interval of filter pores were obtained.

### (Thickness)

A thickness of the filter was measured using a micrometer (Model No.: CLM1-15QMX, manufactured by Mitutoyo Corporation).

### (Total Light Transmittance)

A total light transmittance of the film before the filter pores are formed thereon was measured using a transmittance meter (Model No.: HR-100, manufactured by Murakami Color Research Laboratory Co., Ltd.). A 40 mm × 40 mm film test piece was prepared, and a transmittance of a light beam including all parallel components and diffused components among light beams transmitted by a halogen beam was defined as the total light transmittance.

### (Shore Hardness)

A Shore hardness of the film before the filter pores are formed thereon was measured using a hardness meter (Model NO.: GS719N, manufactured by Teclock Co., Ltd.).

### (Young's Modulus)

A Young's modulus of the film before the filter pores are formed thereon was measured using a tensile tester (Model NO.: RTA-500, manufactured by Orientec Co., Ltd.). Measurement was performed under conditions of a temperature of 23°C, a humidity of 65% RH, and a tensile strength of 300 mm/min.

### [Examples and Comparative Examples]

### (Example 1)

A film mainly containing a polyethylene (melting point: 126°C) and having a thickness of 15 µm was used as a film, and a film mainly containing a cycloolefin polymer (COP) (glass transition temperature: 136°C) and having a thickness of 100 µm was used as a support film. One surface layer of the film has an adhesive layer composed mainly of a low-density polyethylene and having a thickness of 6 µm. The adhesive layer of the film was laminated so as to be attached to a surface of the support film, thereby preparing a laminated structure.

As a mold, a mold in which truncated conical protrusion structures are disposed on an entire surface thereof was used. A truncated cone has a circular bottom surface with a diameter of 8 µm, a height of 50 µm, and is disposed in a square at a pitch of 16 µm. A region where the protrusion structures are processed was a region of 100 mm (film width direction) × 100 mm (film conveying direction). As a material of the mold, copper having a thickness of 20 mm was used as a base material and a nickel plating film was formed on a surface thereof, and a truncated cone pattern was formed on the plating film by machining.

A mold temperature during molding was 150°C, and a pressure of 5 MPa was applied to the entire surface as a pressurizing force. A pressurizing time was 30 seconds. A mold temperature during peeling was 80°C. By peeling off the film from the mold, a thermoplastic film having through holes in the film and recesses communicating with the through holes in the support film was obtained.

The thermoplastic film peeled off from the mold was continuously fed to a winding device, and the film was peeled off from the support film and each of them was wound up. The wound film was obtained as a filter having a plurality of filter pores.

### (Example 2)

A filter was produced by a procedure same as that in Example 1 using a mold same as that in Example 1 except that an arrangement of the protrusion structures was changed such that a porosity rate was 10%.

### (Comparative Example 1)

A Screen Cell (registered trademark) CYTO R kit (Model No.: RCY 4FC) made of a polycarbonate was used as a material.

### (Comparative Example 2)

A Cell Sieve (trade name) Microfilters-8 micron diameter 5 pack (for training) made of an epoxy resin was used as a material.

For the filter in each Example, results of evaluation of each item described above are shown in Table 1. In the table, "N.D." indicates that the corresponding data is not measured. An interval between the filter pores in Comparative Example 1 was evaluated as "undetermined" since there was variation in the value. The filter pores in Examples 1 and 2 and Comparative Example 2 were arranged at equal intervals (substantially equal intervals).

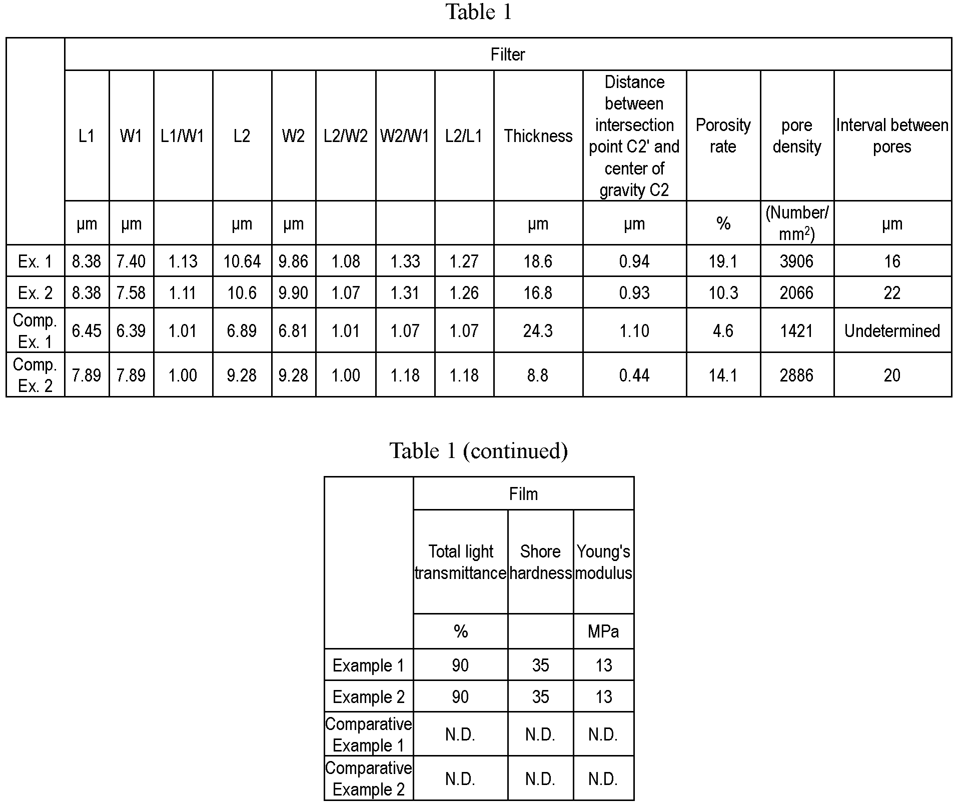

### [Natural Falling Test]

### (Test Method)

For the filter in each Example, it was evaluated whether filtration by natural falling due to an own weight of the specimen was possible. The filter in each Example was set in a filter holder (SWINNEXSX 0001300, manufactured by Millipore Corporation) for a 13 mm diameter filter. A 20 mL syringe (Terumo Syringe 20 mL SS-20ESZ, manufactured by Terumo Corporation) with a plunger removed was set in the filter holder. In the syringe, 5 mL of a physiological saline solution and 5 mL of whole blood were used as specimens, and a time until each filtration was completed was measured. In the case where dropping did not start in 60 seconds, it was determined that filtration by natural falling was difficult, and "no dropping" was shown in the table. For the filter in each Example, using 5 mL of a physiological saline solution and 5 mL of whole blood, the test was respectively performed three times in Example 1 and once in Example 2 and Comparative Examples 1 and 2. The filter that showed "no dropping" in 5 mL of a physiological saline solution was not tested for 5 mL of whole blood since it was determined to be difficult to filter 5 mL of whole blood which has a higher viscosity by natural falling.

Test results are shown in Table 2. Here, when "a to b seconds" is shown in Table 2, "a" represents a minimum number of seconds and "b" represents a maximum number of seconds among the test results performed a plurality of times.

**Table 2**

| | Example 1 | Example 2 | Comparative Example 1 | Comparative Example 2 |
|---|---|---|---|---|
| Physiological saline solution 5 mL | 55 to 75 seconds | No dropping | No dropping | No dropping |
| Whole blood 5 mL | 260 seconds | Not evaluated | Not evaluated | Not evaluated |

### [Cell Collection Rate Test]

### (Preparation of Specimen)

A lung adenocarcinoma cell line H827 was mixed with blood collected from a volunteer such that a concentration was 100/mL, and 3 mL of the mixture was used for one time evaluation and subjected to a cell collection rate evaluation test. This was evaluated as an undiluted specimen. A specimen obtained by diluting the undiluted specimen three times with a physiological saline solution and a specimen obtained by diluting the undiluted specimen five times with a physiological saline solution were also evaluated in the same manner. The evaluation was performed three times using the undiluted specimen (No. 1 to 3), three times using the specimen diluted three times (No. 4 and 3), and three times using the specimen diluted five times (No. 7 to 9).

### (Test Method)

The filter in Example 1 was set in a filter holder and a syringe was set in the same manner as in the natural falling test. Each of the above specimens (No. 1 to 9) was filtered using the syringe.

### (Evaluation)

Cells remained on the filter by separation after filtration (filter-trapped cells) were evaluated for the following items.

Cell measurement value: The filter after filtration was stained with a Giemsa staining solution, the number of tumor cells was counted with a microscope, and the obtained number was defined as a cell measurement value (number). In counting, both a single cell and a cell cluster composed of two or more cells were counted as one.

Collection rate: (Cell measurement value/total number of cells) × 100 was defined as the collection rate (%). The total number of cells was 300 for the undiluted specimen, 100 for the specimen diluted three times, and 60 for the specimen diluted five times.

The number of cell clusters: The number of cell clusters composed of two or more cells among the cell measurement values was defined as the number of cell clusters (number).

The following items were evaluated for a filtrate after separation (a flow through liquid). Half of the filtrate was evaluated by ViCell, and the remaining half was evaluated as a smear specimen.

ViCell: The number of cells was counted using a ViCell XR (BECKMAN manufactured by COULTER) for the half of the filtrate, and the obtained number was defined as the number of ViCell.

Smear specimen: Half of the filtrate was dropped onto a slide glass, smeared by a drawing glass method, and then stained with a Giemsa staining solution. The number of tumor cells was counted with a microscope, and the obtained number was defined as the number of the smear specimen.

The evaluation results are shown in Table 3.

**Table 3**

| | | Filter-trapped cell | | | Flow through liquid | |
|---|---|---|---|---|---|---|
| | N o | Cell measurement value | Collection rate | Number of cell clusters | ViCell | Smear specimen |
| Undiluted | 1 | 14 | 4.7% | 1 | 0 | 3 |
| | 2 | 13 | 4.3% | 0 | 0 | 1 |
| | 3 | 3 | 10% | 0 | 0 | 0 |
| Diluted three times | 4 | 10 | 100% | 0 | 0 | 0 |
| | 5 | 9 | 9.0% | 0 | 0 | 0 |
| | 6 | 8 | 8.0% | 0 | 0 | 0 |
| Diluted five times | 7 | 9 | 150% | 0 | 0 | 0 |
| | 8 | 7 | 11.7% | 0 | 0 | 0 |
| | 9 | 5 | 8.3% | 0 | 0 | 0 |

From the above results, it was confirmed that, according to the filter in Example 1, the rare cells in the blood can be separated by the filter pore having a predetermined pore shape, and the specimen can be filtered without performing the pretreatment such as a hemolysis treatment which may damage the rare cells. Further, it was confirmed that the filter in Example 1 does not require any special equipment such as a suction device, and the filtration of the specimen by natural falling is possible.

Although the cell collection rate test using the filter in Example 2 was not performed, the filter in Example 2 was also produced under conditions same as those in Example 1 except for the arrangement of the filter pores. Similarly, it is considered that the rare cells in the blood can be separated, and the specimen can be filtered without performing the pretreatment such as a hemolysis treatment which may damage the rare cells.

According to the results of the cell collection rate test, the filtrate after separation contained almost no rare cells. Accordingly, it is considered that there are almost no rare cells passing through the filter according to the present invention.

Although the present invention has been described in detail with reference to specific embodiments, it is apparent to those skilled in the art that various changes and modifications can be made without departing from the spirit and scope of the present invention. The present application is based on a Japanese patent application (JP-A-2021-058467) filed on March 30, 2021, the contents of which are incorporated herein by reference.

### Reference Signs List

1 Filter
3 filter pore
100 One surface
103 First opening
106 Region corresponding to first opening
200 The other surface
203 Second opening
206 Region corresponding to second opening
400 Reflector
10 Laminated structure
11 Film
12 Support film
20 Mold
21 Protrusion structure

## Claims

1. A filter having a plurality of filter pores penetrating one surface and the other surface of the filter,
wherein
the filter pore has a first opening on the one surface and a second opening on the other surface,
a ratio (L1/W1) of a major axis diameter L1 to a minor axis diameter W1 of the first opening is 1.00 or more and 1.20 or less,
the minor axis diameter W1 is 7.0 µm or more and 9.0 µm or less,
a ratio (L2/W2) of a major axis diameter L2 to a minor axis diameter W2 of the second opening is 1.00 or more and 1.20 or less, and
a ratio (W2/W1) of the minor axis diameter W2 to the minor axis diameter W1 and a ratio (L2/L1) of the major axis diameter L2 to the major axis diameter L1 are both 1.20 or more and 1.50 or less.

2. The filter according to claim 1, having a thickness of 10 µm or more and 30 µm or less.

3. The filter according to claim 1 or 2, wherein a distance between a center of gravity C2 of the second opening and an intersection point C2' between the other surface and a perpendicular line drawn from a center of gravity C1 of the first opening to the other surface is 3 µm or less.

4. The filter according to any one of claims 1 to 3, having a porosity rate of 10.5% or more and 25% or less.

5. The filter according to any one of claims 1 to 4, wherein the plurality of filter pores are arranged at equal intervals.

6. The filter according to any one of claims 1 to 5, wherein an interval between the filter pores is 8 µm or more and 30 µm or less.

7. The filter according to any one of claims 1 to 6, being a filter comprising a film in which the filter pores are provided, the film having a total light transmittance of 80% or more.

8. The filter according to any one of claims 1 to 7, being a filter comprising a film in which the filter pores are provided, the film having a Shore hardness of 30 or more and 50 or less and a Young's modulus of 5 MPa or more and 25 MPa or less.

9. The filter according to any one of claims 1 to 8, comprising a thermoplastic resin.

10. The filter according to claim 9, wherein a main component of the thermoplastic resin is a polyethylene or a polypropylene.

11. A filter device comprising:
the filter according to any one of claims 1 to 10; and
a holding portion configured to hold the filter, and
the filter device being attachable to and detachable from a syringe.

12. A method for manufacturing the filter according to any one of claims 1 to 10, the method comprising pressing a mold having a protrusion structure on a surface thereof against a film while heating the mold to form a filter pore.

13. A method for separating or fractionating rare cells in a cell suspension, the method comprising a filtration step of filtering a cell suspension using the filter according to any one of claims 1 to 10 or the filter device according to claim 11.

14. The method for separating or fractionating rare cells according to claim 13, wherein in the filtration step, the cell suspension is filtered due to an own weight thereof.

15. The method for separating or fractionating rare cells according to claim 13 or 14, wherein the rare cell is one or more cells selected from the group consisting of a cancer cell, a circulating tumor cell (CTC), an epithelial-mesenchymal transition CTC (EMTCTC), a clustered CTC, a vascular endothelial cell, a vascular endothelial precursor cell, a cancer stem cell, an epithelial cell, a hematopoietic stem cell, a mesenchymal stem cell, a fetal cell, and a combination thereof.

16. The method for separating or fractionating rare cells according to any one of claims 13 to 15, wherein in the filtration step, the cell suspension is filtered from a side of the one surface of the filter.

17. A method for analyzing rare cells in a cell suspension, the method comprising analyzing the rare cells separated or fractionated by the method according to any one of claims 13 to 16 by a method including observing kinetics of the rare cells or measuring activity of the rare cells, or analyzing genes of the rare cells.
